(19) Europäisches Patentamt
European Patent Office
Office européen des brevets

(11) EP 1 154 787 B1

(12) **EUROPEAN PATENT SPECIFICATION**

(45) Date of publication and mention of the grant of the patent:
**10.08.2005 Bulletin 2005/32**

(21) Application number: **00913596.3**

(22) Date of filing: **25.02.2000**

(51) Int Cl.[7]: **A61K 38/21**, A61P 31/14
// A61K38:21, A61K31:365,
A61K38:21, A61K31:4164,
A61K38:21, A61K31:517,
A61K38:21, A61K31:13,
A61K38:21, A61K31:505,
A61K38:21, A61K31:7052

(86) International application number:
**PCT/US2000/004699**

(87) International publication number:
**WO 2000/050064 (31.08.2000 Gazette 2000/35)**

(54) **SYNERGISTIC COMBINATION COMPRISING AN INTERFERON AND A NUCLEOSIDE WITH AN ANTIFLAVIVIRAL AND ANTIRHABDOVIRAL EFFECT**

SYNERGISTISCHE KOMBINATION ENTHALTEND EIN INTERFERON UND EIN NUCLEOSID MIT ANTIFLAVIVIRALEM UND ANTIRHABDOVIRALEM EFFEKT

COMBINAISON SYNERGETIQUE COMPRENANT UN INTERFERON ET UN NUCLEOSIDE AYANT UN EFFET ANTI-FLAVIVIRAL ET ANTI-RHABDOVIRAL

(84) Designated Contracting States:
**AT BE CH CY DE DK ES FI FR GB GR IE IT LI LU MC NL PT SE**

(30) Priority: **26.02.1999 US 121931 P**
**08.02.2000 US 181068 P**

(43) Date of publication of application:
**21.11.2001 Bulletin 2001/47**

(73) Proprietor: **Institute of Molecular and Cell Biology Singapore 117609 (SG)**

(72) Inventors:
- **TAN, Yin, Hwee**
**Singapore 117609 (SG)**
- **DRISCOLL, John, S.**
**Rockville, MD 20854 (US)**

(74) Representative: **Woods, Geoffrey Corlett**
**J.A. KEMP & CO.**
**Gray's Inn**
**14 South Square**
**London WC1R 5JJ (GB)**

(56) References cited:
**EP-A- 0 331 080**
**WO-A-00/15210**
**WO-A-98/19670**
**GB-A- 1 135 898**
**US-A- 4 975 434**
**US-A- 5 380 879**
**US-A- 5 676 942**

- **NEYTS J ET AL: "Use of the yellow fever virus vaccine strain 17D for the study of strategies for the treatment of yellow fever virus infections." ANTIVIRAL RESEARCH, vol. 30, no. 2-3, 1996, pages 125-132, XP000946359 ISSN: 0166-3542**
- **ANDREI G. ET AL: "Molecular approaches for the treatment of hemorrhagic fever virus infections." ANTIVIRAL RESEARCH, (1993) 22/1 (45-75). , XP000971421**
- **HOLY A ET AL: "STRUCTURE-ACTIVITY STUDIES ON OPEN-CHAIN ANALOGUES OF NUCLEOSIDES: INHIBITION OF S-ADENOSYL-L-HOMOCYSTEINE HYDROLASE AND ANTIVIRAL ACTIVITY. 1.NEUTRAL OPEN-CHAIN ANALOGUES" COLLECTION OF CZECHOSLOVAK CHEMICAL COMMUNICATIONS,ACADEMIC PRESS, LONDON,GB, vol. 50, 1985, pages 245-261, XP000960342 ISSN: 0010-0765**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 154 787 B1

- ARROYO J I ET AL: "EFFECT OF HUMAN GAMMA INTERFERON ON YELLOW FEVER VIRUS INFECTION" AMERICAN JOURNAL OF TROPICAL MEDICINE AND HYGIENE, vol. 38, no. 3, 1988, pages 647-650, XP000946332 ISSN: 0002-9637
- DE CLERCQ E ET AL: "ANTIVIRAL ACTIVITIES OF 5-ETHYNYL-1-BETA-D-RIBOFURANOSYLIMIDAZOLE- 4-CARBOXAMIDE AND RELATED COMPOUNDS" ANTIMICROBIAL AGENTS AND CHEMOTHERAPY, vol. 35, no. 4, 1991, pages 679-684, XP000971464 ISSN: 0066-4804
- DRISCOLL J S ET AL: "CYCLOPENTENYL CYTOSINE A NEPLANOCIN ANALOGUE WITH PRECLINICAL ANTITUMOR AND ANTIVIRAL ACTIVITY" PROCEEDINGS AMERICAN ASSOCIATION FOR CANCER RESEARCH ANNUAL MEETING, vol. 28, 1987, page 328 XP000946357 ISSN: 0197-016X
- FOSTER G R ET AL: "DIFFERENT RELATIVE ACTIVITIES OF HUMAN CELL-DERIVED INTERFERON- ALPHA SUBTYPES: IFN-ALPHA8 HAS VERY HIGH ANTIVIRAL POTENCY" JOURNAL OF INTERFERON AND CYTOKINE RESEARCH,US,MARY ANN LIEBERT, NEW YORK, NY, vol. 16, no. 12, 1996, pages 1027-1033, XP000876621 ISSN: 1079-9907
- HYNES JB ET AL: "Quinazolines as inhibitors of dihydrofolate reductase. 4. Classical analogues of folic and isofolic acids" JOURNAL OF MEDICINAL CHEMISTRY, vol. 20, no. 4, 1977, pages 588-591, XP002155519
- J A FINDOR ET AL: "AMANTADINE HCL ALONE AND ASSOCIATED WITH RECOMBINANT ALPHA IFN 2a DURING A SHORT TERM THERAPY IN CHRONIC HCV INFECTION" HEPATOLOGY,US,WILLIAMS AND WILKINS, BALTIMORE, MD, vol. 26, no. 4, 1 October 1997 (1997-10-01), page 217A XP002093941 ISSN: 0270-9139
- LUSCRI B J: "SYNERGISTIC EFFECTS OF HUMAN INTERFERONS WITH EITHER AMANTADINE OR RIMANTADINE" ABSTRACTS OF THE ANNUAL MEETING OF THE AMERICAN SOCIETY FOR, vol. 83, 1983, page A130 XP000946512 1983 ISSN: 0094-8519

## Description

### Field of the Invention

This invention relates to the use of interferons as antiviral agents in the manufacture of a medicament.

### Background to the Invention

Human interferons (IFN) were originally prepared from human white blood cells, a by-product of blood banks, and from primary human fibroblasts in culture. The white cell product was called human leukocyte interferon or human alpha interferon and the human fibroblast interferon was called human β interferon. Subsequent to the cloning of interferons in *E. coli,* several subtypes of human interferons have been identified. There are at least 15 subtypes of alpha interferon (Henco K. *et al*., J. Mol Biol. 185, 227-260, 1985) and only one β interferon.

Cyclopentenyl cytosine (CPE-C) was a promising anti-tumor compound in clinical trial until two deaths were observed in the trial. Four of the eleven patients who received 4.7 mg/$m^2$ per hour of CPE-C experienced hypotension 24 to 48 hours after completion of the CPE-C infusion during their first, third and sixth cycles respectively. Two of the four hypotensive patients died with refractory hypotension. The trial was immediately stopped by the National Cancer Institute, USA. Hypotension was not seen in patients receiving ≤2.5 mg/$m^2$ per h CPE-C (Cancer Chemother. Pharmacol. 36, 513-523, 1995). The mean CPE-C steady-state plasma levels (Cpss) increased linearly from 0.4μM to 3.1μM at doses ranging from I to 5.9 mg/$m^2$/h.

Mycophenolic acid was first demonstrated by H. Florey in 1946 to inhibit bacteria, fungi and leukocytes. The prodrug mycophenolate mofetil is now an FDA-approved transplantation drug.

### Summary of the Invention

We have now found that an interferon such as interferon α2, interferon α8 or interferon β can act synergistically with particular compounds to combat flavivirus and rhabdovirus infections. This synergistic antiviral effect means that lower concentrations of the small molecular weight chemicals such as CPE-C can be used together with interferons as antiflaviral and antirhabdoviral agents and at concentrations below its anticipated side effects (hypotension) in man (1.0 μM or less).

Accordingly, the present invention provides a method of treating a host having a flavivirus or rhabdovirus infection, which method comprises the step of administering to the host in respective amounts which produce a synergistic antiflaviviral or synergistic antirhabdoviral effect:

(a) an interferon, and
b) at least one 5-membered cyclic nucleoside having the formula (I):

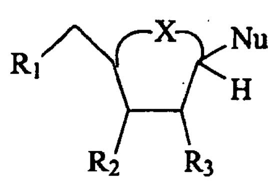

(I)

wherein ⌢ X ⌢ is =CH-, -$CH_2$- or -O-, $R_2$ and $R_3$ are independently selected from the group consisting of H, OH, O-acyl, O-aryl and O-silyl, and $R_1$ is as defined for $R_2$ and $R_3$ or is O-phosphate, Nu is chosen from

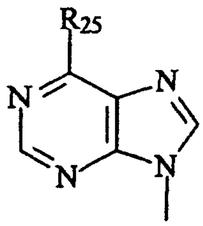
(i)

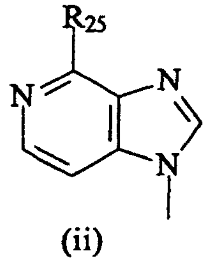
(ii)

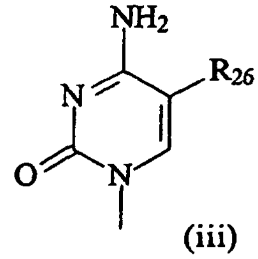
(iii)

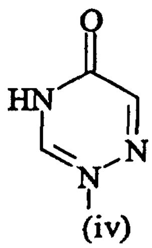
(iv)

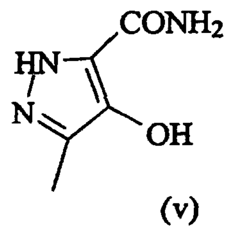
(v)

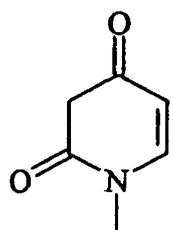
(vi)

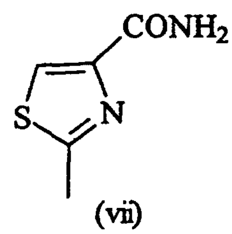
(vii)

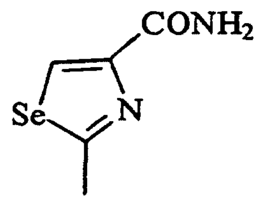
(viii)

and pharmaceutically acceptable metabolites, metabolite derivatives and salts thereof.

The present invention encompasses all optical isomers of the compound (b), in particular the D- and L-isomers of the nucleosides of formula (I).

The invention additionally provides:

- use of an interferon in the manufacture of a medicament for use with at least one compound (b) as defined above in the treatment of a flavivirus or rhabdovirus infection;
- use of at least one compound (b) as defined above in the manufacture of a medicament for use with an interferon in the treatment of a flavivirus or rhabdovirus infection; and
- products containing an interferon and at least one compound (b) as defined above as a combined preparation for simultaneous, separate or sequential use in treating a flavivirus or rhabdovirus infection.

## Detailed Description of the Invention

### Interferons

The interferon for use in the present invention may be an interferon $\alpha$, such as interferon $\alpha 2$ or $\alpha 8$, or interferon $\beta$. The term "interferon" includes fragments which have interferon activity and mutant forms of an interferon which retain interferon activity. For example, the sequence of an interferon $\alpha$ or $\beta$ may have been modified to enhance activity or stability as reported in US-A-5582824, US-A-5593667 or US-A-5594107.

The interferon may have been purified from natural sources or may be a recombinant interferon. The species of interferon is generally the same as the host species to which the interferon is administered. The invention is particularly applicable to the treatment of flavivirus infections in humans. Preferably therefore a human interferon such as

human interferon α2 or α8 or human interferon β is used.

The interferon α8, particularly the human interferon α8, typically has a specific activity of more than $0.6x10^9$, generally from $0.6x10^9$ to $1.5x10^9$ and preferably from $0.8x10^9$ to $1.5x10^9$, IU per mg protein. The human interferon β typically has a specific activity of from $4x10^8$ to $8x10^8$, preferably from $4.8x10^8$ to $6.4x10^8$, IU per mg protein. Interferon α and interferon β specific activities are determined according to reference standards Gb-23-902-530 and Gb-23-902-531 respectively. Specific activity is determined according to a modification of the method of Armstrong, Applied Microbiology 21, 723, 1971, in which 0.2 μg/ml of actinomycin D is included in the viral challenge and the viral induced cytopathic effect (CPE) is read directly from the primary human fibroblasts. Alternatively, specific activity is determined according to the method of Armstrong using WISH cells as assay cells and Encephalomyocarditis virus as challenge virus and the viral induced CPE is read directly.

The interferon such as the interferon α2 or α8 or the interferon β is preferably obtainable by the methodology of WO 96/30531. The interferon is thus obtainable by a process comprising culturing mammalian cells transfected with a nucleic acid vector comprising:

(i) a coding sequence which encodes the interferon and which is operably linked to a promoter capable of directing expression of the coding sequence in mammalian cells in the presence of a heavy metal ion;
(ii) a first selectable marker sequence which comprises a metallothionein gene and which is operably linked to a promoter capable of directing expression of the metallothionein gene in the cells in the presence of a heavy metal ion; and
(iii) a second selectable marker sequence which comprises a *neo* gene and which is operably linked to a promoter capable of directing expression of the *neo* gene in the cells;

unless conditions that allow expression of the coding sequence; and recovering the interferon thus produced.

The transfected mammalian cells may be cells of a human or animal cell line. They may be BHK, COS, CHO Lec2, L CL3, Vero, human fibroblastoid such as C1O, HeLa, or human lymphoblastoid cells or cells of a human tumour cell line. Preferably, however, the cells are CHO cells, particularly wild-type CHO cells.

Desirably, transfected cells will have all or part of such a vector integrated into their genomes. Such cells are preferred because they give stable expression of the coding sequence contained in the vector. Preferably, one or more copies of the entire vector will be integrated, with cells having multiple integrated copies of the vector, for example from 20 to 100 copies or more, being particularly preferred because these cells give a high stable level of expression of the coding sequence contained in the vector.

However, cells having less than complete sections of the vectors integrated into their genomes can be employed if they are functionally equivalent to cells having the entire vector integrated into their genomes, in the sense that the integrated sections of the vector enable the cell to express the coding sequence and to be selected for by the use of heavy metals. Thus, cells exhibiting partial integration of a vector may be employed if the integrated element or elements include the coding sequence operably linked to its associated promoter and the metallothionein marker sequence operably linked to its associated promoter.

Any promoter capable of enhancing expression in a mammalian cell in the presence of a heavy metal ion such as $Cd^{2+}$, $Cu^{2+}$ and $Zn^{2+}$ may be operably linked to the interferon coding sequence. A suitable promoter is a metallothionein gene promoter. The mouse metallothionein gene I (mMT1) promoter is preferred.

Suitable promoter/enhancer combinations for the coding sequence include the mMT1 promoter flanked upstream with a mouse sarcoma virus (MSV) enhancer (MSV-mMT1) and a Rous sarcoma virus (RSV) enhancer upstream of a mouse mammary tumour virus (MMTV) promoter. MSV-mMT1 is preferred.

As far as the first selectable marker sequence is concerned, any promoter capable of enhancing expression in a mammalian cell in the presence of a heavy metal ion such as $Cd^{2+}$, $Cu^{2+}$ and $Zn^{2+}$ may be operably linked to the metallothionein gene such as a human metallothionein gene. Preferably, the marker sequence gene is a human metallothionein gene, such as the human metallothionein gene IIA, which has its own promoter.

The second selectable marker sequence is a *neo* gene. More than one type of this gene exists in nature: any specific *neo* gene can be used in a vector of the invention. One preferred *neo* gene is the *E.coli neo* gene.

The promoter for the *neo* gene is capable of directing expression of the gene in a mammalian cell. Suitable promoters are the cytomegalovirus (CMV) early promoter, the SV40 promoter, the mouse mammary tumour virus promoter, the human elongation factor 1 α-P promoter (EF-1 α-P), the SRα promoter and a metallothionein gene promoter such as mMT1. The promoter may also be capable of expressing the *neo* gene in bacteria such as *E.coli* in which a vector may be constructed.

The interferon coding sequence (i) and the marker sequences (ii) and (iii) are thus each operably linked to a promoter capable of directing expression of the relevant sequence. The term "operably linked" refers to a juxtaposition wherein the promoter and the coding/marker sequence are in a relationship permitting the coding/marker sequence to be expressed under the control of the promoter. Thus, there may be elements such as 5' non-coding sequence

between the promoter and coding/marker sequence. Such sequences can be included in the construct if they enhance or do not impair the correct control of the coding/marker sequence by the promoter.

The vector may be a DNA or RNA vector, preferably a DNA vector. Typically, the vector is a plasmid. Each of the sequences (i) to (iii) will typically be associated with other elements that control their expression. In relation to each sequence, the following elements are generally present, usually in a 5' to 3' arrangement: a promoter for directing expression of the sequence and optionally a regulator of the promoter, a translational start codon, the coding/marker sequence, a polyadenylation signal and a transcriptional terminator.

Further, the vector typically comprises one or more origins of replication, for example a bacterial origin of replication, such as the pBR322 origin, that allows replication in bacterial cells. Alternatively or additionally, one or more eukaryotic origins of replication may be included in the vector so that replication is possible in, for example yeast cells and/or mammalian cells.

The vector may also comprise one or more introns or other non-coding sequences 3' or 5' to the coding sequence or to one or more of the marker sequences. Such non-coding sequences may be derived from any organism, or may be synthetic in nature. Thus, they may have any sequence. Such sequences may be included if they enhance or do not impair correct expression of the coding sequence or marker sequences.

The transfected cells are typically cultured in the presence of a heavy metal ion selected from $Cd^{2+}$, $Cu^{2+}$ and $Zn^{2+}$, particularly in an amount which is not toxic to the cells. That can lead to higher expression of the desired interferon. The concentration of the heavy metal ion in the culture medium is typically from 100 to 200 μM. Cells may therefore be cultured in the presence of from 100 to 200 μM of a heavy metal ion selected from $Cd^{2+}$, $Cu^{2+}$ and $Zn^{2+}$, for example from 130 to 170 μM of the heavy metal ion. A useful concentration is about 150 μM, particularly when the heavy metal ion is $Zn^{2+}$.

The interferon that is produced may be recovered by any suitable means and the method of recovery may vary depending on, for example, the type of cells employed and the culture conditions that have been used. Desirably, the interferon produced will be purified after recovery. Substantially pure interferon can thus be obtained.

The human β-interferon provided by WO 96/30531 has a high degree of sialylation. Like natural human β-interferon produced by primary diploid human fibroblasts, it is well glycosylated. However, it has a higher bioavailability than the natural β-interferon or recombinant β-interferon produced in *E.coli* (betaseron).

The higher bioavailability of the β-interferon can be characterised. When 1.5 x $10^6$ IU of the interferon is injected subcutaneously into the back of a rabbit of about 2 kg: (a) ≥ 128 IU/ml of the interferon is detectable in the serum of the rabbit after 1 hour, and/or (b) ≥ 64 IU/ml of the interferon is detectable in the serum of the rabbit after 5 hours.

The maximum level of interferon is typically observed after 1 hour. According to (a), therefore, 128 to 256 IU/ml such as 140 to 190 IU/ml of the interferon may be detectable in the rabbit serum after I hour. After 5 hours according to (b), ≥ 70 IU/ml such as ≥ 80 IU/ml of the interferon may be detectable in the rabbit serum. Typically according to (b), an amount of interferon in the range of 64 to 128 IU/ml such as 80 to 110 IU/ml can be detected.

Additionally or alternatively, the human interferon β can be characterised by its specific activity. It can have a specific activity from 4.8 x $10^8$ to 6.4 x $10^8$ IU per mg equivalent of bovine serum albumin protein, as noted above. The specific activity may be from 5 x $10^8$ to 6 x $10^8$, for example from 5.2 x $10^8$ to 5.8 x $10^8$ such as from 5.3 x $10^8$ to 5.5 x $10^8$, IU per mg equivalent of bovine serum albumin protein.

The human interferon β may also be characterised by one or more of the following properties:

1. The interferon β typically has an apparent molecular weight of 26,300 as determined by 15% sodium dodecyl sulphate-polyacrylamide gel electrophoresis (SDS-PAGE).
2. When injected as a neat intravenous bolus into a rabbit, the half life of the interferon is typically in the range of from 12 to 15 min such as about 13½ min. The bolus is injected into the rabbit ear vein and blood samples are withdrawn from the rabbit ear artery. Rabbit serum is assayed for the antiviral activity of the interferon according to the modification of the method of Armstrong (1971).
3. The antiviral activity of the interferon in a human hepatoblastoma cell line (HepG2) is at least equal to and, typically, about 1.5 times the activity of natural interferon β from primary diploid human fibroblast cells. The interferon is also about 2.2 times more effective than betaseron in protecting Hep2 cells against a viral challenge. Antiviral activity is again determined according to the modified method of Armstrong (1971). Actinomycin D was omitted in the antiviral determination in HepG2 cells.

The oligosaccharides associated with the interferon β used in the invention may also characterise the interferon β. The interferon β carries oligosaccharides which can be characterised by one or more of the following features:

1. Neutral (no acidic substituents): 5 to 15%, preferably about 10% or lower. Acidic : 95 to 85%, preferably about 90% or higher.
2. The total desialylated oligosaccharide pool is heterogeneous with at least six distinct structural components

present in the pool.

3. Matrix-Assisted Laser Desorption Ionisation - Time of Flight (MALDI-TOF) mass spectrometry and high resolution gel permeation chromatography data are summarised as follows:

| Mass detected | Composition | Calculated Mass | gu equivalent |
|---|---|---|---|
| 1786.2 | 5Hex, 4HexNAc, 1 2AB, Na | 1782 | 11.1 |
| 1929.9 | 5Hex, 1dHex, 4HexNAc, 1 2AB, Na | 1928 | 12.2 |
| 2295.5 | 6Hex, 1dHex, 5HexNAc 1 2AB, Na | 2293 | 14.5 |
| 2660.1 | 7Hex, 1dHex, .6HexNAc 1 2AB, Na | 2658 | 17.6 |
| 3019.1 | 8Hex, 1dHex, 7HexNAc, 1 2AB, Na | 3023 | 20.7 |

The carbohydrate moiety of the human interferon β of WO 96/30531 consists of bi-, tri- and tetra-antennary complex type N-linked oligosaccharides. These oligosaccharides contain repeating lactosamine(s). About 20 to 50%, for example 20 to 30%, 30 to 40% or 35 to 50%, of the oligosaccharides are bi-antennary oligosaccharides. About 30 to 65%, for example from 40 to 60% or 50 to 60%, of the oligosaccharides are tri-antennary oligosaccharides. About 2 to 15%, for example from 2 to 8%, 4 to 10% or 5 to 15%, of the oligosaccharides are tetra-antennary oligosaccharides. Percentages are calculated by weight of total analysable oligosaccharide content.

<u>Compounds (b)</u>

The compounds (b) are capable of inhibiting particular enzymes. Amongst the 5-membered cyclic nucleosides of formula (I), some of those in which ⌢X⌢ is -O-, such as pyrazofurin and 6-azauridine, are inhibitors of orotidylate decarboxylase whilst tiazofurin and selenofurin are inhibitors of inositol monophosphate dehydrogenase (IMPH). Compounds of formula (I) in which ⌢X⌢ is =CH- are typically inhibitors of cytosine triphosphate (CTP) synthetase and S-adenosylhomocysteine hydrolase.

The 5-membered cyclic nucleosides of formula (I) are cyclopentenyl carbocyclic nucleosides in which ⌢X⌢ is =CH-. These have the following structure (I'):

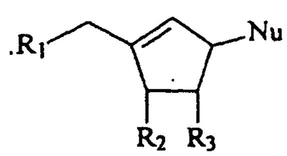

(I').

wherein $R_1$, $R_2$, $R_3$ and Nu are as defined above for formula (I).

The 5-membered cyclic nucleosides of formula (I) and cyclopentenyl carbocyclic nucleosides of formula (I') are compounds in which Nu denotes one of the following groups (i) to (viii):

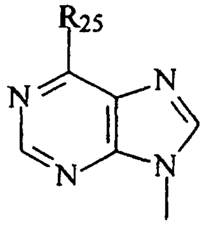

(i)

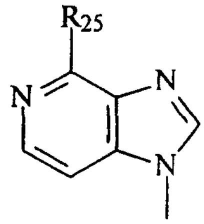

(ii)

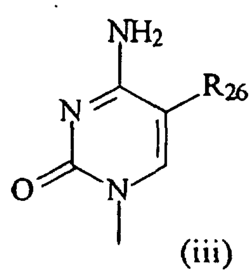

(iii)

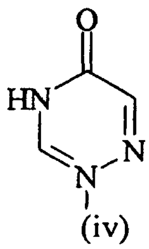

(iv)

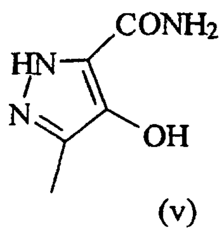

(v)

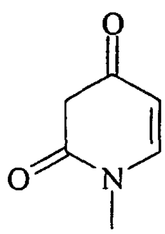

(vi)

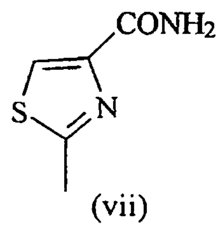

(vii)

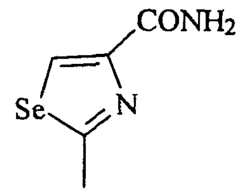

(viii)

wherein $R_{25}$ is Cl or $NH_2$ and $R_{26}$ is H, $CH_3$, $CF_3$, F, Cl, Br or I.

When one or more of $R_1$, $R_2$ and $R_3$ is O-acyl in formula (I) or (I'), the acyl group is preferably a group having the formula $R_{27}$-CO- in which $R_{27}$ is $C_1$-$C_6$ alkyl such as $C_1$-$C_4$ alkyl, for example methyl or ethyl. The O-aryl group is generally a O-($C_6$-$C_{10}$) aryl group such as O-phenyl or O-benzyl. $R_2$ and $R_3$ may together denote a ketal group such as

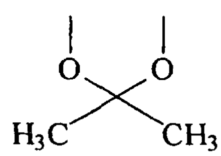

The 5-membered cyclic nucleosides of formula (I) are known compounds and may be synthesised by published procedures or by analogy with published procedures. For instance the synthesis of carbocyclic adenosine [(±)-aristeromycin] is described by Shealy *et al* in J. Am Chem. Soc. 1966, 88, 3885 and in J. Am. Chem. Soc. 1969, 91, 3075. The enantioselective synthesis of (-) aristeromycin and (-)-neplanocin A is described by Arita *et al* in J.Am. Chem. Soc. (1983), 105 (12), 4049-4055.

The synthesis of pyrazofurin is described by Petrie *et al* in J. Med. Chem 1986, 29, 268-278. The synthesis of 6-azauridine is described by Cristescu in Rev. Roum. Chim (1968), 13(3), 365-9. The synthesis of tiazofurin (2-β-D-ribofuranosylthiazole-4-carboxamide) and selenofurin (2-β-D-ribofuranosylselenazole-4-carboxamide) is described by Hennen *et al* in J. Org. Chem (1985) 50(10), 1741-1746. The synthesis of suitable cyclopentenyl carbocyclic nucleosides of formula (I') is described in US-A-4975434. Specific nucleosides are listed in that patent.

The substitution patterns of preferred compounds of formula (I) are shown in Table 1 below. A particularly preferred compound is cyclopentenyl cytosine (CPE-C).

Table 1: Substitution patterns within formula (I)

| ⌢ X ⌢ | $R_1$ | $R_2$ | $R_3$ | formula of Nu | Compound |
|---|---|---|---|---|---|
| =CH - | OH | OH | OH | (ii) wherein $R_{25}$ is $NH_2$ | 3-deazaneplanocin |
| =CH- | OH | OH | OH | (i) wherein $R_{25}$ is $NH_2$ | neplanocin A |
| -O- | OH | OH | OH | (vi) | 3-deazauridine |
| -O- | OH | OH | OH | (iv) | 6-azauridine (NSC 32074) |
| $-CH_2-$ | OH | OH | OH | (i) wherein $R_{25}$ is $NH_2$ | aristeromycin (NSC 103526) |
| -O- | OH | OH | OH | (v) | pyrazofurin (NSC 143095) |
| =CH- | OH | OH | OH | (iii) wherein $R_{26}$ is H | cyclopentenylcytosine (CPE-C) |
| -O- | OH | OH | OH | (vii) | tiazofurin (NSC 286193) |
| -O- | OH | OH | OH | (viii) | selenofurin (NSC 340847) |

As used herein the term "metabolite" refers to a compound for which a compound of formula (I) is a prodrug. For instance, tiaaofurin and selenofurin are prodrugs for thiazolamide adenine dinucleotide (TAD) and selenazolamide adenine dinucleotide (SAD), respectively. In each case the metabolite, rather than the prodrug, is the active moiety. Examples of derivatives (or analogues) of these particular metabolites, which are also encompassed within the present invention, include beta-methylene TAD and beta-methylene SAD. These compounds are published in the literature.

Pharmaceutically acceptable salts, particularly pharmaceutically acceptable acid addition salts, of any of the compounds of formula (I) may be used. Salts with physiologically acceptable inorganic or organic acids may be employed, for example the hydrochloride salt.

Therapeutic uses

The interferons and compounds (b) are used together to treat flavivirus and rhabdovirus infections, particularly in humans. The infection may be acute or chronic. The flavivirus may be yellow fever virus, kunjin virus, dengue virus, hepatitis C virus, or an encephalitis virus such as St. Louis encephalitis virus, Japanese encephalitis virus, Murray valley encephalitis virus and tick-borne encephalitis virus. The rhabdovirus may be vesicular stomatitis virus (VSV) or rabies virus.

The interferon and at least one compound (b) are to be administered to a subject to be treated in sufficient amounts to produce a synergistic antiflaviviral or synergistic antirhabdoviral effect. The condition of the subject can thus be improved. The infection may be cleared from the subject entirely. Relative to monotherapy, lower doses of the interferon and of the compound(s) (b) can be used, which results in a cost-saving and a reduction or elimination of side effects that might occur at higher doses.

Separate formulations of the interferon on the one hand and of the compound(s) (b) on the other hand will generally be given to a patient. A single formulation containing both components can be administered if the interferon and compound(s) (b) are stable in each other's presence and do not otherwise interfere with each other.

The interferon may be administered to humans in various manners such as orally, intravenously, intramuscularly, intraperitoneally, intranasally, intradermally, topically and subcutaneously. The particular mode of administration and dosage regimen will be selected by the attending physician taking into account a number of factors including the age, weight and condition of the patient, the nature of the viral infection, the compound(s) (b) with which it is being administered and the need to obtain a synergistic effect.

The pharmaceutical compositions that contain the interferon as an active principal will normally be formulated with an appropriate pharmaceutically acceptable carrier or diluent depending upon the particular mode of administration being used. For instance, parenteral formulations are usually injectable fluids that use pharmaceutically and physiologically acceptable fluids such as physiological saline, balanced salt solutions, or the like as a vehicle. Oral formulations, on the other hand, may be solids, e.g. tablets or capsules, or liquid solutions or suspensions.

The interferon will usually be formulated as a unit dosage form that contains from $10^4$ to $10^9$, more usually $10^6$ to $10^7$, IU per dose. Typically from 3 x $10^6$ to 18 x $10^6$ IU of interferon is administered per day, particularly by injection such as intravenously or subcutaneously. The dosage may be administered daily, for example for up to five or up to twenty weeks.

The compound(s) (b) can similarly be administered in a variety of dosage forms, for example orally such as in the form of tablets, capsules, sugar- or film-coated tablets, liquid solutions or suspensions, topically such as in the form of creams, ointments or gels, or parenterally, for example intramuscularly, intravenously or subcutaneously. They may therefore be given by injection or infusion.

The mode of administration and dosage regimen of the compound(s) (b) also depends on a variety of factors including the particular compound(s) (b) concerned, the age, weight and condition of the patient, the nature of the viral infection and the need to obtain a synergistic effect. Typically, however, the dosage adopted for each route of administration for humans, for example adult humans, is 0.001 to 10 mg/kg, most commonly in the range of 0.01 to 5 mg/kg, body weight. Such a dosage may be given, for example, daily. The dosage may be given orally or by bolus infusion, infusion over several hours and/or repeated administration.

The interferon and the compound(s) (b) may be given simultaneously. Alternatively they may be given up to five days from each other, for example up to two days apart or up to one day apart or up to four hours apart. The relative timing of the administration of the interferon and the compound(s) (b) may be determined by monitoring their respective serum levels. The interferon may be given before the compound(s) (b) or *vice versa*. Two or three compound(s) (b) may be given, for example simultaneously or spaced apart timewise as above.

A suitable treatment regiment may be illustrated with reference to CPE-C. As noted previously, two patients treated with CPE-C died in a clinical trial as a result of hypotension. At dosages ≤2.5 mg/m$^2$/h no hypotension was observed. Consequently, interferon $\alpha$8 or interferon $\beta$ must be used with CPE-C at dosages of CPE-C below which substantial hyoptension occurs.

This may be achieved according to the invention by subcutaneous or intravenous injection of from 0.01 to 2.5 mg of CPE-C/m$^2$/h and from 3 to 18, for example 3, 6, 12 or 18, x $10^6$ IU of interferon on day 1. The CPE-C may be injected simultaneously with the interferon. Alternatively, the interferon may be injected up to 4 hours later, for example from 1 to 4 hours later. On days 2 and 3, 3 to 18x$10^6$ IU of the interferon may be given to the patient. On day 4, the combination treatment of CPE-C and interferon can be repeated. This regimen may be repeated for several weeks, for example up to five or up to twenty weeks.

The following Examples illustrate the invention.

Example 1

Vero cells were grown to about 99% confluency in 96 microwells with minimum essential medium (MEM) containing 10% fetal calf serum. Growth medium was removed from the microwells and incubated with one of the following. The experiment was done in quadruplicates or duplicates:-

(1) 100µl of interferon α8 containing either 6, 3, 0.6, 0.3, 0.06, or 0.03 IU of interferon (Reference to Gb 23-902-531, NIH standard, distributed by the Natl. Inst. Allergy and Infectious Diseases, NIH, USA) per ml of MEM containing 2.5 mg/ml human serum albumin.
(2) 100µl of interferon α2 containing either 6, 3, 0.6, 0.3, 0.06 or 0.03 IU of interferon (Reference to Gb-23-902-530 as well as Gb-23-902-531 standards) per ml of MEM containing 2.5 mg/ml human serum albumin.
(3) 100µl of interferon β containing 60, 30, 6, 3 or 0.6 IU of interferon (Reference to Gb23-902-531 standard) per ml of MEM containing 2.5 mg/ml human serum albumin.

After the addition of interferon to the Vero cells in the microwell, 100µl of a viral challenge consisting of 100 $TCLD_{50}$ yellow fever virus, kunjin virus or dengue virus was added immediately to each of the microwells. Different concentrations of test compound were included in the viral challenge as well. In the case where no test compound was used (i.e. the viral control), only 100µl of the viral challenge was added to the wells in 100 µl of MEM containing 2.5 mg/ml human serum albumin.

The test compounds used were cyclopentenyl cytosine (CPE-C) from the National Cancer Institute, USA; mycophenolic acid from Sigma Chemicals; 5-ethynyl-1-β-D-ribofuranosylimidazole-4-carboxamide (EICAR); and amantadine hydrochloride.

In the control cells treated with test compound alone, 100 µl of MEM containing 2.5 mg/ml human serum albumin instead of interferon was added to the cells followed by an addition of another 100 µl of MEM/human serum albumin (2.5 mg/ml) containing test compound. In the case of CPE-C, the concentrations of CPE-C in MEM were 2 µM, 0.22 µM and 0.022 µM. The subsequent concentrations of CPE-C in the wells were therefore 1 µM, 0.11 µM and 0.011 µM.

The cells were examined for virus-induced cythopathic effects on day 5, day 8 and day 9 respectively for the antiviral activities of interferon or of test compound(s) or of interferon and test compound(s) protecting against a challenge virus of yellow fever, kunjin or dengue virus. The results of the enhancement of the antiviral effect of interferon by CPE-C is given in Table 3.

Table 3

| Human interferon | µM CPE-C | Fold enhancement of antiviral activity interferon |
|---|---|---|
| α8 | 1 | 30 |
| α8 | 0.11 | 10 |
| α8 | 0.01 | 0 |
| None | 1* | 0 |
| None | 0.11** | 0 |
| None | 0.011# | 0 |
| α2 | 1 | 10 |
| α2 | 0.11 | 5 |
| α2 | 0.01 | 0 |
| None | 1* | 0 |
| None | 0.11** | 0 |
| None | 0.011# | 0 |
| β | 1 | 10 |
| β | 0.11 | 5 |
| β | 0.01 | 0 |
| None | 1* | 0 |
| None | 0.11** | 0 |

* 75-90% of the Vero cells were protected against a yellow fever virus challenge of 100 $TCLD_{50}$
** 40-50% of the Vero cells were protected against a yellow fever virus challenge of 100 $TCLD_{50}$.
# none of the Vero cells were protected against a yellow fever virus challenge of 100 $TCLD_{50}$ at this concentration of CPE-C.

Table 3 (continued)

| Human interferon | μM CPE-C | Fold enhancement of antiviral activity interferon |
|---|---|---|
| None | 0.011# | 0 |

Similar results in enhancement of the antiviral activity of interferon were observed when a viral challenge of 100 $TCLD_{50}$ of kunjin virus (0.11 μM of CPE-C enhanced interferon α8, interferon α2 and interferon β by 10-, 5- and 5-fold respectively) or dengue virus (0.11 μM of CPE-C enhanced interferon α8, interferon α2 and interferon β by 12-, 5- and 6-fold respectively) was used in place of yellow fever virus.

Similar experiments were repeated with mycophenolic acid, amantadine hydrochloride and EICAR. The concentration required to enhance the anti-viral activity of human interferon α8 is summarized in Table 4.

Table 4

| Human interferon | μM of test compounds # | Fold enhancement of antiviral activity | |
|---|---|---|---|
| α8 | 0.1 CPE-C(C) | 10 | I |
| α8 | 0.1 mycophenolic acid | 3 | C |
| α8 | (MA) | 3 | C |
| α8 | 1.0 EICAR (E) | 2 | C |
| α8 | 1.0 amantadine (A) | 50 | C |
| where I = inventive and C = comparative | | | |

# Indicates the concentration of test compound alone to protect Vero cells against a viral challenge by approximately 50%. In the case of mycophenolic acid, 2% fetal calf serum was used in place of human serum albumin.

Example 2

Monkey (Vero) or human (HuH7) cells were grown on 96 microwells as described in Example 1. When the cells were grown to 90-100% confluency with growth medium (MEM or DMEM) containing 5 or 10% fetal calf serum, the growth medium was removed from the microwells and the cells re-incubated with one of the following. The experiment was done in quadruplicates or duplicates:-

> 100 μl of human interferon α8, α2 or β interferon serially diluted from 30, 10, 3.3, 1.0, 0.33, 0.1, 0.033, 0.01, etc (Reference to Gb 23-902-531, NIH standard, distributed by the Natl. Inst. Allergy and Infectious Diseases, NIH, USA). In certain experiments using human cells, a serial 2-fold dilution of the interferon was used instead of the 30, 10, 3.3, 1.0, etc, dilution steps.

After adding the different concentrations of interferon to the cells in the microwells, 100 μl of a viral challenge (yellow fever, dengue, kunjin virus or VSV) was immediately added to the microwells of cells previously treated with interferon and such that the viral challenge has a final $TCLD_{50}$ of 100. Different concentrations of test compounds were added in the viral challenge. In the case where no test compound was used (viral control) 100 μl viral challenge was added to the wells in 100 μl of MEM containing 2.5 mg/ml human serum albumin. The interferon control cells were challenged with virus without test compound. The test compound controls were challenged with virus but no interferon. The test compounds used were cyclopentenyl cytosine (CPE-C) from the National Cancer Institute, USA; mycophenolic acid from Sigma Aldrich Inc.;. 5-ethynyl-1-β-D-ribofuranosylimidazole-4-carboxamide (EICAR); amantadine hydrochloride; neplanocin A (provided by NCI, U.S.A.); 3-deazaneplanocin A (provided by NCI, USA); 3-deazauridine (Aldrich Chemical Co Inc.); NSC 382046, NSC 143095 and Artisteromycin (Sigma Aldrich Inc).

The cells were examined for virus-induced cytopathic effects on days 1, 2, 3, 4, 5, 6, 7 and 8, depending on the virus used for the antiviral activities of interferon or of test compound(s) or of interferon and test compound(s) protecting against a challenge virus of either yellow fever, dengue, kunjin virus or VSV. The results of the enhancement of the antiviral effect of the interferon used by each of the above test compounds are shown in the following Tables 5 to 22.

In the tables the $IC_{50}$ values represent the concentration of each agent required to give a 50% inhibition of cell death following viral challenge. Where an $IC_{50}$ value is shown as zero for one agent, that agent was not present. In those cases the $IC_{50}$ given for the other agent is the $ED_{50}$ of the latter alone.

CI in the tables represents the combination index. This is a parameter which reflects the presence or absence of synergism between two pharmacologically active agents. The calculation of the combination index is described by T.M. Brennan *et al* in Antiviral Research 1995, 173-187. The equation as used in the present experiments was therefore

$$CI = \left( \frac{\text{concentration of drug (b)}}{\text{IC}_{50} \text{ of drug (b) alone}} \right) + \left( \frac{\text{concentration of IFN}}{\text{IC}_{50} \text{ of IFN alone}} \right)$$

If the CI is equal to I the two agents have a mutually additive effect. If the CI is less than 1 they have a synergistic effect. If the CI is greater than 1 they have a mutually antagonistic effect. (M.C. Berenbaum in Pharmacol. Rev. 41, 93-141 (1989)).

Table 5: Mycophenolic acid (MPA) and IFN in Vero cells (Comparative Example)

| Virus | IFN α2 $IC_{50}$ (IU/ml) | MPA $IC_{50}$ (μM) | CI | IFN α8 $IC_{50}$ (IU/ml) | MPA $IC_{50}$ (μM) | CI | IFN β $IC_{50}$ (IU/ml) | MPA $IC_{50}$ (μM) | CI |
|---|---|---|---|---|---|---|---|---|---|
| Yellow fever | 0.153 | 0 | - | 0.127 | 0 | - | 4.688 | 0 | - |
| | 0.076 | 0.275 | 0.695 | 0.076 | 0.184 | 0.731 | 1.376 | 1.388 | 0.96 |
| | 0.076 | 0.413 | 0.794 | 0.048 | 0.275 | 0.576 | 0 | 2.081 | - |
| | 0.048 | 0.619 | 0.757 | 0.048 | 0.413 | 0.675 | | | |
| | 0.048 | 0.928 | 0.980 | 0.048 | 0.619 | 0.822 | | | |
| | 0 | 1.388 | - | 0 | 1.388 | | | | |
| Dengue virus | 0.489 | 0 | - | 0.489 | 0 | - | 4.688 | 0 | - |
| | 0.244 | 0.275 | 0.702 | 0.244 | 0.275 | 0.702 | 1.465 | 0.413 | 0.61 |
| | 0.153 | 0.413 | 0.610 | 0.076 | 0.413 | 0.453 | 1.465 | 0.619 | 0.758 |
| | 0.015 | 0.619 | 0.474 | 0.048 | 0.619 | 0.542 | 0.458 | 0.928 | 0.764 |
| | 0.015 | 0.928 | 0.697 | 0.016 | 0.928 | 0.699 | 0 | 1.388 | - |
| | 0 | 1.388 | - | 0 | 1.388 | - | | | |

| | | | | | | | | | |
|---|---|---|---|---|---|---|---|---|---|
| Kunjin virus | 0.092 | 0 | - | 0.092 | 0 | - | 0.916 | 0 | - |
| | 0.014 | 0.413 | 0.598 | 0.009 | 0.275 | 0.395 | 0.286 | 0.413 | 0.610 |
| | 0.009 | 0.619 | 0.767 | 0.009 | 0.413 | 0.544 | 0.090 | 0.619 | 0.542 |
| | 0 | 0.928 | - | 0.009 | 0.619 | 0.767 | 0.286 | 0.928 | 0.979 |
| | | | | 0 | 0.928 | - | 0 | 1.388 | - |

Table 6: CPE-C and IFN in Vero cells

| Virus | IFN α2 $IC_{50}$ (IU/ml) | CPE-C $IC_{50}$ (μM) | CI | IFN α8 $IC_{50}$ (IU/ml) | CPE-C $IC_{50}$ (μM) | CI | IFN β $IC_{50}$ (IU/ml) | CPE-C $IC_{50}$ (μM) | CI |
|---|---|---|---|---|---|---|---|---|---|
| Yellow Fever | 0.489 | 0 | - | 0.489 | 0 | - | 4.688 | 0 | - |
| | 0.153 | 0.017 | 0.513 | 0.153 | 0.017 | 0.513 | 1.465 | 0.025 | 0.513 |
| | 0.048 | 0.025 | 0.398 | 0.153 | 0.025 | 0.613 | 1.465 | 0.038 | 0.613 |
| | 0.048 | 0.038 | 0.548 | 0.048 | 0.038 | 0.548 | 0.458 | 0.056 | 0.531 |
| | 0.024 | 0.056 | 0.699 | 0.048 | 0.056 | 0.748 | 0.045 | 0.085 | 0.676 |
| | 0 | 0.085 | - | 0 | 0.085 | - | | 0.127 | - |

| | | | | | | | | | | |
|---|---|---|---|---|---|---|---|---|---|---|
| Dengue virus | 4.688 | 0 | - | 1.465 | 0 | - | 30 | 0 | - |
| | 0.229 | 0.142 | 0.144 | 0.458 | 0.017 | 0.345 | 2.930 | 0.285 | 0.231 |
| | 0.045 | 0.199 | 0.141 | 0.143 | 0.033 | 0.162 | 2.930 | 0.398 | 0.284 |
| | 0.045 | 0.279 | 0.197 | 0.045 | 0.067 | 0.159 | 0.916 | 0.558 | 0.291 |
| | 0.045 | 0.390 | 0.270 | 0.045 | 0.134 | 0.279 | 0.916 | 0.781 | 0.395 |
| | 0.045 | 0.547 | 0.374 | 0.045 | 0.268 | 0.535 | 0.286 | 1.093 | 0.519 |
| | 0.045 | 0.765 | 0.519 | 0 | 0.536 | - | 0.286 | 1.530 | 0.723 |
| | 0.045 | 1.071 | 0.724 | | | | 0 | 2.142 | - |
| | 0 | 1.500 | - | | | | | | |
| Kunjin virus | 0.293 | 0 | - | 0.293 | 0 | - | 0.916 | 0 | - |
| | 0.092 | 0.025 | 0.450 | 0.092 | 0.025 | 0.404 | 0.286 | 0.025 | 0.402 |
| | 0.092 | 0.038 | 0.519 | 0.029 | 0.038 | 0.233 | 0.286 | 0.038 | 0.447 |
| | 0.029 | 0.056 | 0.394 | 0.029 | 0.056 | 0.293 | 0.090 | 0.056 | 0.292 |
| | 0.009 | 0.085 | 0.485 | 0.029 | 0.085 | 0.397 | 0.045 | 0.085 | 0.348 |
| | 0.009 | 0.127 | 0.713 | 0.029 | 0.127 | 0.547 | 0.090 | 0127 | 0.546 |
| | 0 | 0.191 | - | 0.029 | 0.191 | 0.756 | 0 | 0.280 | - |
| | | | | 0 | 0.286 | - | | | |

Table 7: 3-deazauridine (3-DAU) and IFN in Vero cells

| Virus | IFN α2 $IC_{50}$ (IU/ml) | 3-DAU $IC_{50}$ (µM) | CI | IFN α8 $IC_{50}$ (IU/ml) | 3-DAU $IC_{50}$ (µM) | CI | IFN β $IC_{50}$ (IU/ml) | 3-DAU $IC_{50}$ (µM) | CI |
|---|---|---|---|---|---|---|---|---|---|
| Yellow Fever | 0.489 | 0 | - | 0.245 | 0 | | 1.465 | 0 | |
| | 0.153 | 4.115 | 0.513 | 0.048 | 12.346 | 0.624 | 0.458 | 20.996 | 0.823 |
| | 0.077 | 12.346 | 0.757 | 0.015 | 20.576 | 0.776 | 0.143 | 29.394 | 0.812 |
| | 0 | 20.576 | - | 0 | 28.807 | | 0 | 41.152 | |

Table 8: 3-deazaneplanocin A (3-DAN) and IFN in Vero cells

| Virus | IFN α2 $IC_{50}$ (IU/ml) | 3-DAN $IC_{50}$ (μM) | CI | IFN α8 $IC_{50}$ (IU/ml) | 3-DAN $IC_{50}$ (μM) | CI | IFN β $IC_{50}$ (IU/ml) | 3-DAN $IC_{50}$ (μM) | CI |
|---|---|---|---|---|---|---|---|---|---|
| Yellow fever | 0.488 | 0 | - | 0.488 | 0 | - | 1.465 | 0 | - |
| | 0.015 | 0.725 | 0.395 | 0.076 | 0.725 | 0.416 | 0.143 | 0.725 | 0.462 |
| | 0.015 | 1.015 | 0.541 | 0.048 | 1.015 | 0.463 | 0.143 | 1.015 | 0.608 |
| | 0.015 | 1.420 | 0.745 | 0.015 | 1.420 | 0.541 | 0.143 | 1.420 | 0.812 |
| | 0 | 1.989 | - | 0 | 1.989 | - | 0 | 1.989 | - |
| Dengue virus | 1.465 | 0 | - | 1.465 | 0 | - | 9.375 | 0 | - |
| | 0.458 | 0.725 | 0.677 | 0.143 | 0.725 | 0.462 | 0.915 | 1.015 | 0.608 |
| | 0.143 | 1.015 | 0.608 | 0.045 | 1.015 | 0.541 | 0.089 | 1.420 | 0.724 |
| | 0.045 | 1.420 | 0.745 | 0.045 | 1.420 | 0.745 | 0 | 1.989 | - |
| | 0 | 1.989 | - | 0 | 1.989 | - | | | |

Table 9: 6-azauridine (6-AU) and IFN in Vero cells

| Virus | IFN α2 $IC_{50}$ (IU/ml) | 6-AU $IC_{50}$ (μM) | CI | IFN α8 $IC_{50}$ (IU/ml) | 6-AU $IC_{50}$ (μM) | CI | IFN β $IC_{50}$ (IU/ml) | 6-AU $IC_{50}$ (μM) | CI |
|---|---|---|---|---|---|---|---|---|---|
| Yellow fever | 0.153 | 0 | - | 0.321 | 0 | - | 2.271 | 0 | - |
| | 0.048 | 1.224 | 0.811 | 0.153 | 0.816 | 0.762 | 1.465 | 0.816 | 0.931 |
| | 0.015 | 1.633 | 0.765 | 0.048 | 1.224 | 0.578 | 0.458 | 1.224 | 0.630 |
| | 0.015 | 2.041 | 0.932 | 0.048 | 1.633 | 0.721 | 0.458 | 1.633 | 0.773 |
| | 0 | 2.449 | - | 0.015 | 2.041 | 0.761 | 0.143 | 2.041 | 0.777 |
| | | | | 0.048 | 2.449 | 1.007 | 0.458 | 2.449 | 1.059 |
| | | | | 0 | 2.857 | - | 0 | 2.857 | - |

Table 10:

| NSC 382046 and IFN in Vero cells against Dengue virus (Comparative Example) | | |
|---|---|---|
| IFN α2 $IC_{50}$ (IU/ml) | NSC 382046 $IC_{50}$ (μM) | CI |
| 4.688 | 0 | - |
| 1.465 | 0.095 | 0.758 |
| 0.045 | 0.141 | 0.672 |
| 0 | 0.212 | - |

Table 11:

| NSC 7364 and IFN in Vero cells against Dengue virus (Comparative Example) | | | | | |
|---|---|---|---|---|---|
| IFN α2 $IC_{50}$ (IU/ml) | NSC 7364 $IC_{50}$ (μM) | CI | IFN α8 $IC_{50}$ (IU/ml) | NSC 7364 $IC_{50}$ (μM) | CI |
| 1.465 | 0 | - | 4.688 | 0 | - |
| 0.733 | 0.186 | 0.834 | 1.465 | 0.372 | 0.813 |
| 0.458 | 0.372 | 0.979 | 0 | 0.743 | - |
| 0 | 0.558 | - | | | |

Table 12: NSC 143095 and IFN in Vero cells against yellow fever virus (Comparative Example)

| IFN α 2 $IC_{50}$ (IU/ml) | NSC 143095 $IC_{50}$ (μM) | CI | IFN α 8 $IC_{50}$ (IU/ml) | NSC 143095 $IC_{50}$ (μM) | CI |
|---|---|---|---|---|---|
| 0.458 | 0 | - | 0.153 | 0 | - |
| 0.143 | 0.848 | 0.756 | 0.048 | 0.386 | 0.813 |
| 0.045 | 1.272 | 0.763 | 0 | 0.772 | - |
| 0 | 1.907 | - | | | |

| IFN β $IC_{50}$ (IU/ml) | NSC 143095 $IC_{50}$ (μM) | CI |
|---|---|---|
| 2.930 | 0 | - |
| 0.287 | 1.272 | 0.764 |
| 0 | 1.907 | - |

Table 13:

| Mycophenolic acid (MPA) and IFN α8 in human cells against VSV and yellow fever virus (Comparative Example) | | | | |
|---|---|---|---|---|
| Virus | Cell type | IFN α8 $IC_{50}$ (IU/ml) | MPA $IC_{50}$ (μM) | CI |
| VSV | MRC 5 | 0 | 4.1 | - |
| | | 4.9 | 0 | - |
| | | 0.02 | 3.9 | 0.96 |
| | | 0.01 | 2.6 | 0.64 |
| | | 0.01 | 1.7 | 0.43 |
| | | 0.21 | 1.2 | 0.33 |
| | | 0.43 | 0.8 | 0.28 |
| | | 1.22 | 0.5 | 0.37 |
| | | 2.18 | 0.3 | 0.53 |
| | | 4.39 | 0.2 | 0.95 |
| | | 4.65 | 0.2 | 0.98 |
| | HuH7 | 0 | 1.72 | - |
| | | 2.06 | 0 | - |
| | | 0.13 | 0.72 | 0.48 |
| | | 0.38 | 0.48 | 0.46 |
| | | 0.77 | 0.32 | 0.56 |
| Yellow fever | HuH7 | 0 | 3.86 | - |
| | | 7.85 | 0 | - |
| | | 1.73 | 2.48 | 0.86 |
| | | 1.94 | 1.65 | 0.68 |
| | | 1.94 | 1.10 | 0.53 |
| | | 1.22 | 0.73 | 0.35 |
| | | 2.76 | 0.49 | 0.48 |
| | | 6.99 | 0.33 | 0.97 |

Table 14:

| CPE-C and IFN in human cells against VSV and kunjin virus. | | | | |
|---|---|---|---|---|
| Cell type | Virus | IFN β $IC_{50}$ (IU/ml) | CPE-C $IC_{50}$ (μM) | CI |
| HuH7 | VSV | 0 | 4.18 | - |
| | | 35.7 | 0 | - |
| | | 0.81 | 1.84 | 0.46 |
| | | 0.81 | 1.23 | 0.32 |
| | | 0.81 | 0.82 | 0.22 |
| | | 1.08 | 0.55 | 0.16 |
| | | 1.94 | 0.36 | 0.14 |
| | | 2.59 | 0.24 | 0.13 |
| | Kunjin | 0 | 0.37 | - |
| | | 2.59 | 0 | - |
| | | 0.20 | 0.25 | 0.75 |
| | | 0.23 | 0.17 | 0.54 |
| | | 0.08 | 0.11 | 0.33 |
| | | 0.81 | 0.07 | 0.51 |
| | | 1.83 | 0.01 | 0.84 |
| Cell type | Virus | IFN α8 $IC_{50}$ (IU/ml) | CPE-C $IC_{50}$ (μM) | CI |
| MRC-5 | VSV | 0 | 2.26 | - |
| | | 3.10 | 0 | - |
| | | 0.19 | 2.09 | 0.98 |
| | | 0.61 | 1.39 | 0.81 |
| | | 1.22 | 0.93 | 0.80 |
| | | 2.45 | 0.41 | 0.97 |
| | | 1.94 | 0.28 | 0.75 |
| | | 2.18 | 0.18 | 0.79 |
| | | 2.18 | 0.12 | 0.76 |
| | | 2.18 | 0.08 | 0.74 |

Table 15:

| 3-deazaneplanocin A (3-DAN) and IFN in human cells against VSV | | | | |
|---|---|---|---|---|
| Cell type | 3-DAN | IFN $IC_{50}$ (IU/ml) | | CI |
| | | TFN α8 | IFN β | |
| MRC 5 | 0.172 | 0 | | - |
| | 0 | 3.10 | | - |
| | 0.115 | 0.38 | | 0.79 |
| | 0.076 | 1.09 | | 0.80 |
| | 0.051 | 1.94 | | 0.92 |
| | 0.034 | 2.18 | | 0.90 |
| | 0.023 | 2.18 | | 0.84 |
| | 0.015 | 2.45 | | 0.88 |
| | 0.010 | 2.45 | | 0.85 |
| HuH7 | 0.126 | | 0 | - |
| | 0 | | 19.80 | - |
| | 0.095 | | 0.54 | 0.79 |
| | 0.064 | | 0.54 | 0.54 |
| | 0.042 | | 0.86 | 0.38 |
| | 0.028 | | 1.22 | 0.29 |
| | 0.019 | | 1.94 | 0.25 |
| | 0.013 | | 2.18 | 0.21 |

Table 16:

| 6-azauridine (6-AU) and IFNβ in human cells against VSV | | | |
|---|---|---|---|
| Cell type | IFN β $IC_{50}$ (IU/ml) | 6-AU $IC_{50}$ (μM) | CI |
| MRC 5 | 0 | 80.8 | - |
| | 2.20 | 0 | - |
| | 0.38 | 25.3 | 0.49 |
| | 1.09 | 7.8 | 0.59 |
| | 1.22 | 2.4 | 0.59 |

Table 17:

| NSC 382046 and IFNβ in human HuH7 cells against yellow fever and kunjin virus (Comparative Example) | | | |
|---|---|---|---|
| Virus | IFN β $IC_{50}$ (IU/ml) | NSC 382046 $IC_{50}$ (μM) | CI |
| Yellow fever | 0 | 0.350 | - |
| | 2.59 | 0 | - |
| | 0.81 | 0.192 | 0.86 |
| | 0.72 | 0.128 | 0.64 |
| | 0.57 | 0.085 | 0.46 |
| | 0.57 | 0.057 | 0.38 |
| | 0.57 | 0.038 | 0.33 |
| | 0.72 | 0.025 | 0.35 |
| | 0.81 | 0.017 | 0.36 |
| | 0.81 | 0.011 | 0.34 |
| Kunjin virus | 0 | 5.264 | - |
| | 18.74 | 0 | - |
| | 8.30 | 2.865 | 0.99 |
| | 11.10 | 1.910 | 0.96 |
| | 11.10 | 1.273 | 0.83 |
| | 11.10 | 0.849 | 0.75 |
| | 11.10 | 0.566 | 0.70 |

Table 18:

| NSC 143095 and IFNβ in human HuH7 cells against yellow fever virus and VSV. (Comparative Example) | | | |
|---|---|---|---|
| Virus | IFN β $IC_{50}$ (IU/ml) | NSC 143095 $IC_{50}$ (μM) | CI |
| VSV | 0 | 11.822 | - |
| | 6.21 | 0.00 | - |
| | 0.08 | 6.448 | 0.56 |
| | 0.08 | 4.299 | 0.38 |
| | 0.25 | 2.866 | 0.28 |
| | 0.57 | 1.910 | 0.25 |
| | 1.83 | 1.274 | 0.40 |
| | 3.68 | 0.849 | 0.66 |
| Yellow fever | 0 | 0.510 | - |
| | 2.06 | 0.00 | - |
| | 0.08 | 0.340 | 0.71 |
| | 0.25 | 0.227 | 0.57 |
| | 0.81 | 0.151 | 0.69 |

Table 19:

| Tiazofurin and IFNβ in human HuH7 cells against yellow fever virus and kunjin virus | | | |
|---|---|---|---|
| Virus | IFNβ $IC_{50}$ (IU/ml) | Tiazofurin $IC_{50}$ (μM) | CI |
| Yellow fever | 0 | 7.538 | - |
| | 0.37 | 0 | - |
| | 0.01 | 6.154 | 0.85 |
| | 0.05 | 4.103 | 0.68 |
| | 0.10 | 1.823 | 0.51 |
| | 0.28 | 0.812 | 0.87 |
| Kunjin virus | 0 | 6.923 | - |
| | 0.20 | 0 | - |
| | 0.01 | 4.615 | 0.73 |
| | 0.01 | 3.077 | 0.51 |
| | 0.02 | 2.051 | 0.38 |
| | 0.03 | 1.368 | 0.32 |
| | 0.04 | 0.912 | 0.33 |
| | 0.04 | 0.608 | 0.29 |
| | 0.08 | 0.405 | 0.46 |

Table 20:

| Selenofurin and IFNβ in human HuH7 cells against yellow fever virus and kunjin virus | | | |
|---|---|---|---|
| Virus | IFNβ $IC_{50}$ (IU/ml) | Selenofurin $IC_{50}$ (μM) | CI |
| Yellow fever | 0 | 0.472 | - |
| | 0.43 | 0 | - |
| | 0.05 | 0.391 | 0.94 |
| | 0.04 | 0.261 | 0.65 |
| | 0.07 | 0.173 | 0.53 |
| | 0.16 | 0.117 | 0.62 |
| | 0.16 | 0.052 | 0.49 |
| Kunjun virus | 0 | 0.717 | - |
| | 0.20 | 0 | - |
| | 0.01 | 0.554 | 0.84 |
| | 0.01 | 0.369 | 0.58 |
| | 0.05 | 0.164 | 0.48 |
| | 0.05 | 0.109 | 0.40 |
| | 0.08 | 0.073 | 0.51 |
| | 0.08 | 0.049 | 0.47 |

Table 21:

| Amantadine and human IFN in human cells against VSV and kunjin virus (Comparative Example) | | | | | |
|---|---|---|---|---|---|
| Cell Type | Virus | Amantadine $IC_{50}$ (μM) | IFN $IC_{50}$ (IU/ml) | | CI |
| | | | IFN α8 | IFN β | |
| MRC-5 | VSV | 913.8 | 0 | | - |
| | | 0 | 0.81 | | - |
| | | 584.9 | 0.12 | | 0.79 |
| | | 390.1 | 0.27 | | 0.76 |
| | | 259.9 | 0.27 | | 0.62 |
| | | 173.0 | 0.38 | | 0.66 |
| | | 115.8 | 0.61 | | 0.87 |
| HuH7 | Kunjin virus | 115.7 | | 0 | |
| | | 0 | | 0.13 | |
| | | 77 | | 0.01 | 0.76 |
| | | 51.3 | | 0.04 | 0.76 |

Table 22:

| Neplanocin A and human IFN in human cells against VSV and Yellow Fever Virus | | | | | |
|---|---|---|---|---|---|
| Cell Type | Virus | Neplanocin A $IC_{50}$ (μM) | IFN $IC_{50}$ (IU/ml) | | CI |
| | | | IFN α8 | IFN β | |
| MRC-5 | VSV | 2.9 | 0 | | - |
| | | 0 | 2.18 | | - |
| | | 2.2 | 0.04 | | 0.80 |
| | | 1.5 | 0.09 | | 0.56 |
| | | 1.0 | 0.12 | | 0.40 |
| | | 0.7 | 0.97 | | 0.67 |
| | | 0.4 | 1.22 | | 0.71 |
| HuH7 | Yellow Fever | 9.3 | | 0 | |
| | | 0 | | 5.86 | |
| | | 5.1 | | 0.01 | 0.63 |
| | | 3.4 | | 0.04 | 0.71 |
| | | 2.2 | | 2.06 | 0.59 |

Example 3 (Comparative Example)

By following the experimental procedure described in Examples I and 2 the compound NSC 184692 was found to have an $ED_{50}$ against dengue virus of 120 pM in monkey Vero cells subjected to viral challenge by 100 $TCLD_{50}$ dengue virus. It was found that 100 pM of the compound enhanced the anti-viral effect of human IFNβ 10-fold.

## Claims

1. Use, in the manufacture of a medicament for the treatment of a flavivirus or rhabdovirus infection, of:

    (a) an interferon, and
    (b) at least one 5-membered cyclic nucleoside having the formula (I):

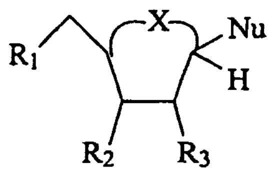

(I)

wherein $\frown$ X $\frown$ is =CH-, -$CH_2$- or -O-, $R_2$ and $R_3$ are independently selected from the group consisting of H, OH, O-acyl, O-aryl and O-silyl, and $R_1$ is as defined for $R_2$ and $R_3$ or is O-phosphate, Nu is chosen from

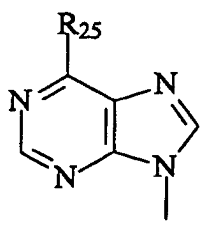

(i)

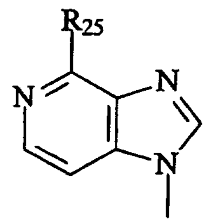

(ii)

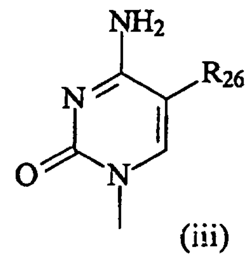

(iii)

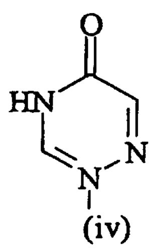

(iv)

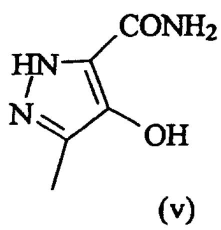

(v)

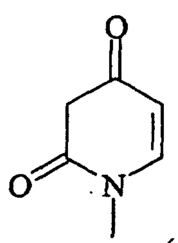

(vi)

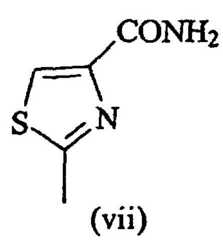

(vii)

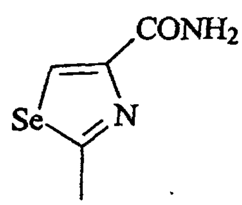

(viii)

wherein $R_{25}$ is Cl or $NH_2$ and $R_{26}$ is H, $CH_3$, $CF_3$, F, Cl, Br or I, and pharmaceutically acceptable metabolites, metabolite derivatives and salts thereof.

2. Use of an interferon in the manufacture of a medicament for use with at least one compound (b) as defined in claim 1 in the treatment of a flavivirus or rhabdovirus infection.

**3.** Use of at least one compound (b) as defined in claim 1 in the manufacture of a medicament for use with an interferon in the treatment of flavivirus or rhabdovirus infection.

**4.** Use according to any one of claims 1 to 3, wherein the flavivirus is selected from yellow fever virus, kunjin virus, dengue virus, hepatitis C virus, St. Louis encephalitis virus, Japanese encephalitis virus, Murray valley encephalitis virus and tick-borne encephalitis virus.

**5.** Use according to any one of claims 1 to 3, wherein the rhabdovirus is selected from vesicular stomatitis virus (VSV) and rabies virus.

**6.** Use according to any one of claims I to 3, wherein the interferon (a) is a human interferon.

**7.** Use according to any one of claims 1 to 3, wherein the interferon is selected from interferon $\alpha 2$, interferon $\alpha 8$ and interferon $\beta$.

**8.** Use according to claim 7, wherein the interferon is human interferon $\alpha 8$ having a specific activity of from $0.6 \times 10^9$ to $1.5 \times 10^9$ IU per mg protein.

**9.** Use according to claim 7, wherein the interferon is human interferon $\beta$ having a specific activity of from $4 \times 10^8$ to $8 \times 10^8$ per mg protein.

**10.** Use according to any one of the preceding claims wherein the compound (b) is at least one compound selected from cyclopentenyl cytosine, 3-deazaneplanocin, neplanocin A, 3-deazauridine, 6-azauridine, aristeromycin, pyrazofurin, tiazafurin and selenofurin.

**11.** Products containing an interferon and at least one compound (b) as defined in claim 1 as a combined preparation for simultaneous, separate or sequential use in treating a flavivirus or rhabdovirus infection.

**12.** An agent for use in the treatment of a flavivirus or rhabdovirus infection, which comprises an interferon and at least one compound (b) as defined in claim 1.

## Patentansprüche

**1.** Verwendung bei der Herstellung eines Medikaments zur Behandlung einer Flavivirus- oder Rhabdovirusinfektion von:

(a) einem Interferon, und
(b) mindestens einem 5-gliedrigen cyclischen Nucleosid mit der Formel (I):

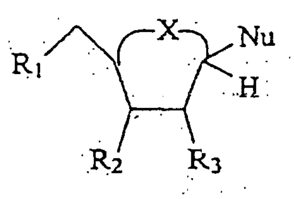

(I)

worin ⌒X⌒ bedeutet =CH-, $-CH_2-$ oder -O-, $R_2$ und $R_3$ unabhängig voneinander ausgewählt sind aus der Gruppe, bestehend aus H, OH, O-Acyl, O-Aryl und O-Silyl, und worin $R_1$ wie für $R_2$ und $R_3$ definiert ist oder O-Phosphat darstellt, Nu ausgewählt ist unter

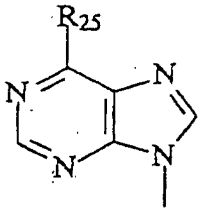
(i)
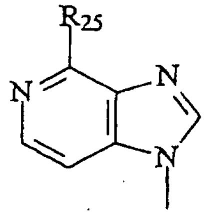
(ii)
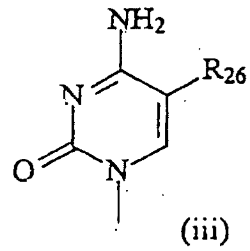
(iii)

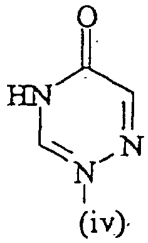
(iv)
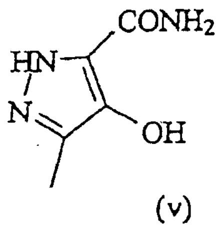
(v)

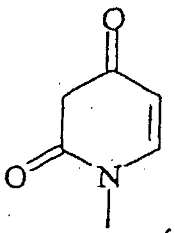
(vi)
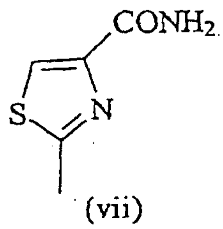
(vii)
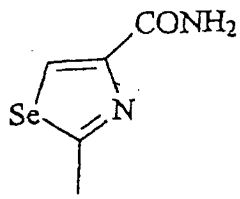
(viii)

worin $R_{25}$ Cl oder $NH_2$ bedeutet und $R_{26}$ H, $CH_3$, $CF_3$, F, Cl, Br oder I ist, sowie von pharmazeutisch annehmbaren Metaboliten, Metabolitderivaten und Salzen derselben.

2. Verwendung eines Interferons bei der Herstellung eines Medikaments zur Verwendung mit mindestens einer Verbindung (b), wie in Anspruch 1 definiert, bei der Behandlung einer Flavivirus- oder Rhabdovirusinfektion.

3. Verwendung mindestens einer Verbindung (b), wie in Anspruch 1 definiert, bei der Herstellung eines Medikaments zur Verwendung mit einem Interferon bei der Behandlung einer Flavivirus- oder Rhabdovirusinfektion.

4. Verwendung nach einem der Ansprüche 1 bis 3; worin der Flavivirus ausgewählt ist unter dem Gelbfiebervirus, Kunjinvirus, Dengue-Virus, Hepatitis C-Virus, St. Louis-Enzephalitis-Virus, dem japanischen Enzephalitis-Virus, dem Murray-Valley-Enzephalitis-Virus und dem von Zecken übertragenen Enzephalitis-Virus.

5. Verwendung nach einem der Ansprüche 1 bis 3, worin der Rhabdovirus ausgewählt ist unter dem vesiculären Stomatitisvirus (VSV) und dem Tollwutvirus.

6. Verwendung nach einem der Ansprüche 1 bis 3, worin das Interferon (a) ein humanes Interferon ist.

7. Verwendung nach einem der Ansprüche 1 bis 3, worin das Interferon ausgewählt ist unter Interferon $\alpha 2$, Interferon $\alpha 8$ und Interferon $\beta$.

8. Verwendung nach Anspruch 7, worin das Interferon humanes Interferon $\alpha 8$ mit einer spezifischen Aktivität von $0{,}6 \times 10^9$ bis $1{,}5 \times 10^9$ IU pro mg Protein ist.

**9.** Verwendung nach Anspruch 7, worin das Interferon menschliches Interferon β mit einer spezifischen Aktivität von 4 x $10^8$ bis 8 x $10^8$ IU pro mg Protein ist.

**10.** Verwendung nach einem der vorstehenden Ansprüche, worin die Verbindung (b) mindestens eine Verbindung ist, ausgewählt unter Cyclopentenylcytosin, 3-Desazaneplanocin, Neplanoxin A, 3-Desazauridin, 6-Azauridin, Aristeromycin, Pyrazofurin, Tiazafurin und Selenofurin.

**11.** Produkte, enthaltend ein Interferon und mindestens eine Verbindung (b) wie in Anspruch 1 definiert, als Kombinationspräparat für die gleichzeitige, separate oder sequentielle Verwendung bei der Behandlung einer Flavivirus- oder Rhabdovirusinfektion.

**12.** Mittel zur Verwendung bei der Behandlung einer Flavivirus- oder Rhabdovirusinfektion, das ein Interferon und mindestens eine Verbindung (b) enthält, wie in Anspruch 1 definiert.

## Revendications

**1.** Utilisation, dans la fabrication d'un médicament destiné au traitement d'une infection à flavivirus ou à rhabdovirus, de :

(a) un interféron, et
(b) au moins un nucléoside cyclique à 5 chaînons ayant la formule (I) :

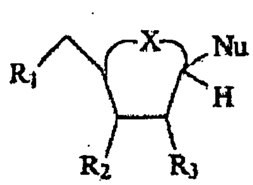

(I)

dans laquelle ⌢X⌢ représente =CH-, -$CH_2$- ou -O-, $R_2$ et $R_3$ sont choisis indépendamment dans le groupe constitué par H, OH, les groupes O-acyle, O-aryle et O-silyle, et $R_1$ est tel que défini pour $R_2$ et $R_3$ ou représente un groupe O-phosphate, Nu est choisi parmi

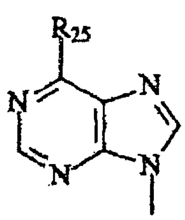

(i)

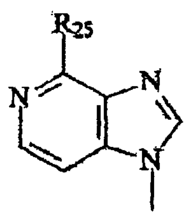

(ii)

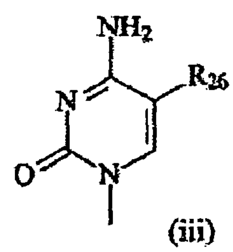

(iii)

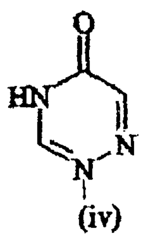

(iv)

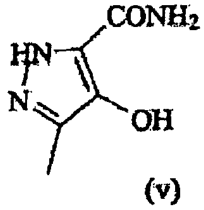

(v)

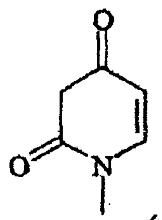 (vi) 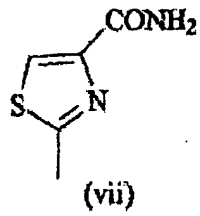 (vii) 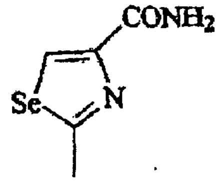 (viii)

dans lesquelles $R_{25}$ représente Cl ou $NH_2$ et $R_{26}$ représente H, $CH_3$, $CF_3$, F, Cl, Br ou I et des métabolites, des dérivés de métabolites et des sels pharmaceutiquement acceptables de ceux-ci.

**2.** Utilisation d'un interféron dans la fabrication d'un médicament pour utilisation avec au moins un composé (b) tel que défini dans la revendication 1 dans le traitement d'une infection à flavivirus ou à rhabdovirus.

**3.** Utilisation d'au moins un composé (b) tel que défini dans la revendication 1 dans la fabrication d'un médicament pour utilisation avec un interféron dans le traitement d'une infection à flavivirus ou à rhabdovirus.

**4.** Utilisation selon l'une quelconque des revendications 1 à 3, dans laquelle le flavivirus est choisi parmi le virus de la fièvre jaune, le virus Kunjin, le virus de la dengue, le virus de l'hépatite C, le virus de l'encéphalite de Saint-Louis, le virus de l'encéphalite japonaise, le virus de l'encéphalite de la vallée de Murray et le virus de l'encéphalite à tique.

**5.** Utilisation selon l'une quelconque des revendications 1 à 3, dans laquelle le rhabdovirus est choisi parmi le virus de la stomatite vésiculaire (VSV) et le virus de la rage.

**6.** Utilisation selon l'une quelconque des revendications 1 à 3, dans laquelle l'interféron (a) est un interféron humain.

**7.** Utilisation selon l'une quelconque des revendications 1 à 3, dans laquelle l'interféron est choisi parmi l'interféron $\alpha 2$, l'interféron $\alpha 8$ et l'interféron $\beta$.

**8.** Utilisation selon la revendication 7, dans laquelle l'interféron est l'interféron $\alpha 8$ humain ayant une activité spécifique de $0,6 \times 10^9$ à $1,5 \times 10^9$ UI par mg de protéine.

**9.** Utilisation selon la revendication 7, dans laquelle l'interféron est l'interféron $\beta$ humain ayant une activité spécifique de $4 \times 10^8$ à $8 \times 10^8$ par mg de protéine.

**10.** Utilisation selon l'une quelconque des revendications précédentes, dans laquelle le composé (b) est au moins un composé choisi parmi la cyclopenténTyl-cytosine, la 3-déazanéplanocine, la néplanocine A, la 3-déazauridine, la 6-azauridine, l'aristéromycine, la pyrazofurine, la tiazafurine et la sélénofurine.

**11.** Produits contenant un interféron et au moins un composé (b) tel que défini dans la revendication 1 sous la forme d'une préparation combinée pour une utilisation simultanée, séparée ou séquentielle dans le traitement d'une infection à flavivirus ou à rhabdovirus.

**12.** Agent pour utilisation dans le traitement d'une infection à flavivirus ou à rhabdovirus, qui comprend un interféron et au moins un composé (b) tel que défini dans la revendication 1.